# FASCICULE DE BREVET EUROPEEN

(11) **EP 0 935 976 B1**
(45) Date de publication et mention de la délivrance du brevet: **17.12.2003**
(21) Numéro de dépôt: 99400257.4
(22) Date de dépôt: 04.02.1999
(51) Int. Cl.: A61M 25/00

(54) **Tige flexible biocompatible destinée à être implantée dans un conduit anatomique et dispositif équipé d'une telle tige**
Flexibler, biokompatibler Schaft zur Implantation in einen Körpergang und eine mit diesem Schaft ausgerüstete Vorrichtung
Flexible biocompatible shaft to be implanted into a body duct and device fitted with such a shaft

(30) Priorité: 16.02.1998 FR 9801836
(43) Date de publication de la demande: 18.08.1999
(73) Titulaire: B. Braun Medical SAS, 92100 Boulogne Billancourt (FR)
(72) Inventeur: Nadal, Guy, 86000 Poitiers (FR); Chevillon, Gérard, 92120 Montrouge (FR)
(74) Mandataire: Lerner, François

(56) Documents cités:
- EP-A- 0 530 970
- EP-A- 0 680 734
- EP-A- 0 747 022
- WO-A-93/15785
- US-A- 5 573 521

## Description

La présente invention concerne la mise en place à l'intérieur d'un corps humain d'une tige flexible de préférence creuse (mais éventuellement pleine), et en particulier l'introduction d'une telle tige à l'intérieur d'un conduit anatomique.

Il est considéré que ce type de tige présente actuellement certains inconvénients et ne donne pas entière satisfaction, aussi bien au praticien qui la manipule qu'au patient à l'intérieur du corps duquel elle est placée. En particulier, cette tige n'offre pas une flexibilité appropriée pour que l'on puisse la manipuler aisément, sans danger et sans douleur pour le patient. Elle n'offre donc pas un compromis rigidité/souplesse satisfaisant avec un certain degré d'élasticité.

La présente invention propose de résoudre une partie au moins de ces problèmes.

Pour cela, il est proposé que cette tige flexible comprenne une structure hélicoïdale à spires non jointives sur une partie au moins de sa longueur. Dans une application particulière, une telle tige est utilisée dans un dispositif pour l'introduction d'un implant, tel qu'une prothèse ou implant expansible (stent, prothèse pour anévrisme, filtre sanguin) comprenant une gaine externe flexible ainsi que la tige présentée ci-avant montée à l'intérieur de la gaine sur une partie au moins de sa longueur de telle sorte que ladite gaine et la tige puissent coulisser longitudinalement l'une par rapport à l'autre pour, dans une première position relative, maintenir l'implant dans un premier état radialement restreint à l'intérieur de la gaine, et, dans une seconde position relative, permettre à l'implant d'être libéré hors de la gaine dans le conduit dans un second état radialement déployé.

Pour permettre une bonne manipulation de l'implant et notamment pour lui procurer un appui arrière suffisamment rigide, sans pour autant nuire à la flexibilité de la tige-poussoir, la structure hélicoïdale de la tige s'étendra de préférence jusqu'à proximité de son extrémité distale sans l'atteindre, la tige se terminant alors de préférence à cette extrémité, là où elle vient en appui contre l'implant, par une partie pleine.

Pour des questions d'hygiène du praticien qui utilise le dispositif d'implantation, et afin de faciliter la prise en main du dispositif lors de la mise en place de l'implant de l'extérieur du corps du patient, il sera préférable que la structure hélicoïdale de la tige s'étende jusqu'à proximité de son extrémité proximale sans l'atteindre, de manière que cette extrémité proximale puisse présenter une paroi périphérique continûment pleine sur la partie de la longueur de la tige qui est située hors de la gaine externe dans ladite première position relative de la tige et de la gaine.

Pour contrôler encore plus précisément la flexibilité de la tige, ladite tige pourra comprendre, sur une partie au moins de sa longueur, une suite de portions à structure hélicoïdale alternée(s) avec une(des) portion(s) à surface périphérique continûment pleine.

Dans le même souci et si nécessaire pour permettre la circulation d'un liquide, voire le passage d'un guide-fil, la tige pourra par ailleurs être doublée intérieurement, sur l'essentiel au moins de sa longueur, par un cathéter.

Selon une considération complémentaire, ce cathéter s'étendra de préférence jusqu'au-delà de l'extrémité distale de la tige et sera entouré, à l'écart de celle-ci, par un embout profilé de façon à définir une cavité de chargement de l'implant située entre l'extrémité distale de la tige et l'embout. Cette solution sera particulièrement adaptée à l'implantation d'une endoprothèse expansible préchargée vers l'extrémité distale de son dispositif d'implantation.

Selon une considération complémentaire, l'extrémité distale du cathéter se termine à l'intérieur de l'embout profilé et est prolongé par une portion tubulaire qui s'étend au-delà de l'extrémité effilée de l'embout et qui est plus flexible que ledit cathéter.

Enfin, toujours dans le but d'optimiser la flexibilité de la tige, l'interstice vide entre deux spires de la structure hélicoïdale de la tige présentera, dans un état non comprimé axialement de celle-ci, une longueur moyenne axiale comprise entre environ 0,1 et 1 millimètre mm et le pas des spires de cette structure hélicoïdale sera de préférence compris entre environ 1 et 5 millimètres.

L'invention et sa mise en oeuvre apparaîtront encore plus clairement à l'aide de la description qui va suivre, faite en référence aux dessins dans lesquels :
- la figure 1 est une vue d'un dispositif conforme à l'invention destiné à l'implantation d'une prothèse autoexpansible dans un vaisseau sanguin d'un patient,
- la figure 2 est une vue en coupe de l'extrémité distale du dispositif de la figure 1, la prothèse étant chargée dans celui-ci,
- la figure 3 est une vue en coupe de l'extrémité distale du dispositif de la figure 1, la prothèse étant sur le point d'être implantée dans le vaisseau du patient,
- la figure 4 est une vue en coupe de l'extrémité distale du dispositif de la figure 1 une fois la prothèse totalement déployée,

Le dispositif introducteur de la présente invention est repéré 10 dans son ensemble. Il comprend tout d'abord une gaine externe 20 flexible biocompatible présentant une extrémité proximale 22 et une extrémité distale 24.

Le dispositif 10 comprend également une tige poussoir 30 flexible et biocompatible, de diamètre externe légèrement inférieur au diamètre interne de la gaine 20 pour pouvoir coulisser à l'intérieur de celle-ci. Ladite tige 30 est ici creuse, présente un axe général longitudinal xx' ainsi qu'une extrémité proximale 32 et une extrémité distale 34.

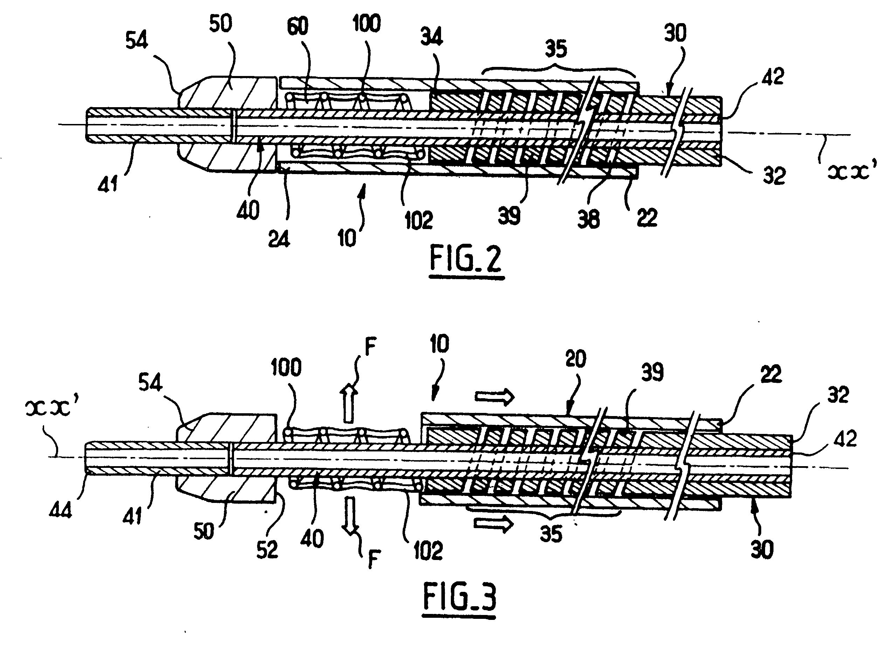

Le dispositif peut aussi comprendre un cathéter 40 de renfort dont le diamètre externe est sensiblement égal au diamètre interne de ladite tige poussoir 30. Ce cathéter 40 passe au travers de la tige poussoir 30 sur toute sa longueur et est solidarisé à l'extrémité proximale 32 de celle-ci par exemple par un manchon serti (non représenté). L'extrémité distale 44 du cathéter 40 ressort au-delà de l'extrémité distale 34 de la tige. A distance de la tige 30, un embout 50 profilé, de diamètre externe sensiblement égal à celui de la tige et comprenant une extrémité distale 54 profilée et une extrémité proximale 52, est serti autour de l'extrémité distale 44 du cathéter 40 et définit, avec l'extrémité distale 34 de la tige, une cavité 60 (ou zone de chargement) pour une endoprothèse 100 recouverte d'une enveloppe souple 102 préchargée dans un état radialement contraint (voir figure 2). Le cathéter 40 est prolongé, jusqu'à son extrémité distale 44, par une partie tubulaire 41 anti-traumatisante plus courte et plus flexible que lui, sertie à l'intérieur de l'embout 50.

Comme on peut le voir sur la figure 2, la tige poussoir 30 comprend, sur une partie au moins de sa longueur, une structure hélicoïdale 35 à spires 39 non jointives au moins dans un état non contraint axialement, ces spires définissant entre elles des interstices 38 susceptibles de laisser passer un fluide corporel à travers eux. La tige n'est ainsi recouverte d'aucun enrobage interne et/ou externe tel qu'un élastomère ou du silicone, de telle sorte qu'à l'endroit des spires, la tige est exclusivement constituée par cette structure hélicoïdale.

Dans le cas représenté, la structure hélicoïdale 35 est comprise entre l'extrémité distale 34 et l'extrémité proximale 32, à l'exclusion des deux portions d'extrémité. On notera en particulier que la surface périphérique de la portion proximale 32 de la tige poussoir 30 est continûment pleine sur quelques millimètres selon l'axe xx', c'est-à-dire que la tige est dépourvue proche de son extrémité proximale de spires espacées susceptibles de laisser passer un fluide corporel à travers elles. Ce choix est dû notamment au fait que le praticien, lorsqu'il implante la prothèse 100 dans le corps du patient, agit directement sur cette extrémité proximale 32 en la saisissant à pleines mains. Il faut donc une bonne prise en main et éviter toute remonté du fluide corporel jusqu'à cette extrémité.

De même, la surface périphérique de la portion distale 34 du poussoir 30 est également continûment pleine sur quelques millimètres selon l'axe xx' de manière à assurer de bonnes conditions d'appui lors de la mise en place de l'implant dans le corps du patient.

Les étapes d'implantation de l'endoprothèse 100 sont décrites ci-après, en relation avec les figures 2 à 4.

Sur la figure 2, le dispositif 10 est en position d'introduction. Auparavant, il a été introduit par voie endoluminale transcutanée connue (méthode de "SELDINGER") via une voie d'accès aménagée par exemple dans l'artère fémorale ou jugulaire (selon la zone d'implantation) du patient La gaine 20 recouvre donc la cavité 60 à l'intérieur de laquelle l'endoprothèse 100 a été préchargée dans un état radialement restreint.

Sur la figure 3, la cavité 60 n'est plus recouverte par ladite gaine 20 qui a été retirée vers l'arrière par rapport à la tige poussoir 30.
L'endoprothèse 100 peut alors être libérée, par expansion radiale, comme l'indiquent les flèches F de la figure 3.

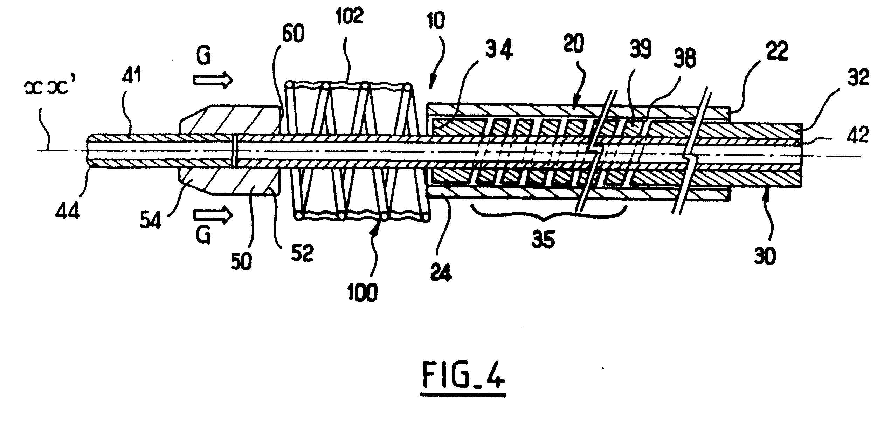

Sur la figure 4, la prothèse 100 est déployée radialement à l'intérieur du conduit anatomique, par exemple à l'endroit d'un anévrisme d'un vaisseau sanguin, de telle sorte qu'il est alors possible de retirer le dispositif 10 à travers le passage intérieur qu'elle présente dans cette position déployée.

La tige 30 permet ainsi en particulier d'éviter les problèmes de plicatures ou de boucles. Elle améliore la flexibilité du dispositif, c'est-à-dire sa capacité à se courber, sans pour autant diminuer sa rigidité axiale indispensable pour permettre de faire sortir l'implant 100 hors de la gaine (notamment au niveau de son extrémité distale 34).

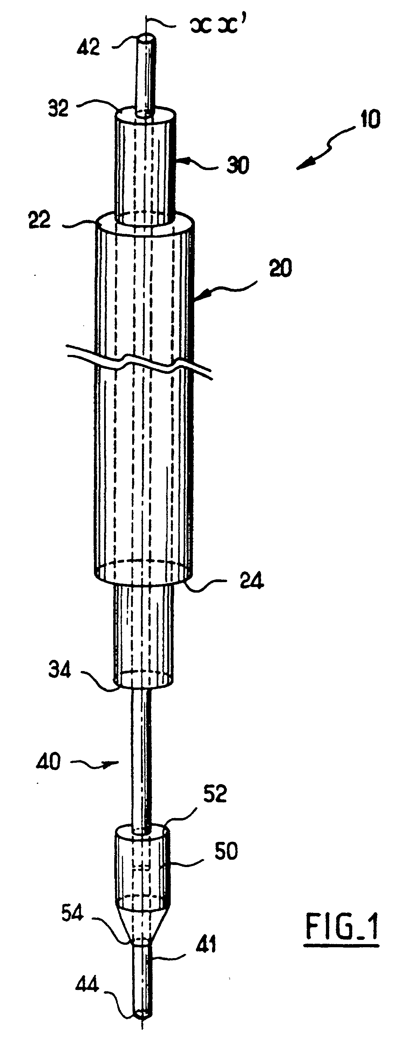

Dans les différents cas représentés sur les figures 1 à 4, le pas P des spires 39 de(s) portion(s) de tige 30 à structure 35 hélicoïdale est de préférence compris entre environ 1 et 5 millimètres, et la longueur axiale moyenne L des interstices 38 est de préférence comprise entre 0,1 et 1 millimètre de façon à procurer une bonne flexibilité à la tige poussoir 30.

Bien entendu, l'invention n'est nullement limitée aux modes de réalisation préférentiels illustrés à titre d'exemple.

Ainsi, le dispositif 10 tel que représenté sur les figures 1 à 4 peut être utilisé pour l'implantation de tout type de prothèse (auto expansible, expansible sur ballon) préchargée dans une cavité prévue à cet effet, ou simplement disposée au bout de la tige 30.

La tige 30 des figures 1 à 4 peut aussi comprendre une série de portions à structure hélicoïdale alternées avec des portions à surface périphérique continûment pleine. Ce type de structure alternée peut être appliquée sur toute la longueur de la tige poussoir, ou entre ses extrémités distale et proximale.

## Revendications

1. Dispositif (10) pour l'introduction d'un implant (100) dans un conduit anatomique (V), ledit dispositif (10) comprenant :
- une gaine (20) externe flexible ayant une extrémité distale (24) et une extrémité proximale (22),
- une tige (30) creuse flexible présentant un axe longitudinal (xx'), une longueur selon cet axe, une extrémité distale (34) destinée à venir en appui contre l'implant et une extrémité proximale (32), ladite tige (30) étant montée glissante à l'intérieur de la gaine (20) sur une partie au moins de sa longueur,
- la gaine (20) externe et la tige (30) pouvant coulisser longitudinalement l'une par rapport à l'autre pour, dans une première position relative, maintenir l'implant (100) dans un premier état radialement restreint à l'intérieur de la gaine (20), et, dans une seconde position relative, permettre à l'implant (100) d'être libéré hors de la gaine (20), dans le conduit (V), dans un second état radialement déployé,
**caractérisé en ce que** la tige (30) comprend, sur une partie au moins de sa longueur, une structure (35) hélicoïdale à spires (39) non jointives présentant, entre elles, des interstices vides (38) susceptibles de laisser passer un fluide corporel.

2. Dispositif (10) selon la revendication 1, **caractérisé en ce que** la structure (35) hélicoïdale de la tige (30) s'étend jusqu'à proximité de son extrémité distale (34) sans l'atteindre, la tige se terminant à cette extrémité (34), là où elle vient en appui contre l'implant (100), par une partie pleine.

3. Dispositif (10) selon l'une quelconque des revendications précédentes, **caractérisé en ce que** la structure (35) hélicoïdale de la tige (30) s'étend jusqu'à proximité de son extrémité proximale (32) sans l'atteindre, de manière que cette extrémité proximale présente une paroi périphérique continûment pleine sur la partie de la longueur de la tige qui est située hors de la gaine externe (20) dans ladite première position relative de la tige et de la gaine.

4. Dispositif (10) selon l'une quelconque des revendications précédentes, **caractérisé en ce que** la tige (30) comprend, sur une partie au moins de sa longueur, une suite de portions (35) à structure hélicoïdale alternées avec une(des) portion(s) (36) à surface périphérique continûment pleine.

5. Dispositif (10) selon l'une quelconque des revendications précédentes, **caractérisé en ce que** la tige (30) est doublée intérieurement, sur l'essentiel au moins de sa longueur par un cathéter (40).

6. Dispositif (10) selon la revendication 5, **caractérisé en ce que** le cathéter (40) s'étend jusqu'au-delà de l'extrémité distale (34) de la tige (30) et est entouré, à l'écart de celle-ci, par un embout (50) profilé de façon à définir une cavité (60) de chargement de l'implant (100) située entre l'extrémité distale (34) de la tige et ledit embout (50).

7. Dispositif (10) selon la revendication 6, **caractérisé en ce que** l'extrémité distale (44) du cathéter (40) se termine à l'intérieur de l'embout (50) profilé et est prolongée par une portion tubulaire (41) anti-traumatisante qui s'étend au-delà de l'extrémité effilée de l'embout (50) et qui est plus flexible que ledit cathéter (40).

8. Dispositif (10) selon l'une quelconque des revendications précédentes, **caractérisé en ce que** l'interstice vide (38) entre deux spires (39) de la structure hélicoïdale (35) de la tige (30) présente, dans un état non comprimé axialement de celle-ci, une longueur moyenne axiale comprise entre environ 0,1 et 1 millimètre.

9. Dispositif selon l'une quelconque des revendications précédentes, **caractérisé en ce que** le pas des spires (39) de la structure hélicoïdale (35) de la tige (30) est compris entre environ 1 et 5 millimètres.

## Patentansprüche

1. Vorrichtung (10) zur Einführung eines Implantates (100) in einen Körper (V), wobei die Vorrichtung (10) aufweist:
- eine äußere, flexible Hülle (20) mit einem distalen Ende (24) und einem proximalen Ende (22),
- einen hohlen, flexiblen Schaft (30), der eine Längsachse (xx'), eine Länge entlang dieser Achse, ein distales Ende (34), das dazu bestimmt ist, gegen das Implantat in Abstützung zu kommen, und ein proximales Ende (32) aufweist, wobei der Schaft (30) gleitend im Inneren der Hülle (20) mindestens an einem Teil ihrer Länge angebracht ist,
- wobei sich die Hülle (20) und der Schaft (30) längs zueinander verschieben lassen, um in einer ersten relativen Position das Implantat (100) in einem ersten radial eingeengten Zustand im Inneren der Hülle (20) zu halten, und in einer zweiten relativen Position zu gestatten, daß das Implantat (100) aus der Hülle (20) in den Körper (V) in einem zweiten radial entfalteten Zustand freigegeben wird,
**dadurch gekennzeichnet, daß** der Schaft (30) mindestens auf einem Teil seiner Länge einen spiralförmigen Aufbau (35) mit nicht aneinanderstoßenden Windungen (39) aufweist, die untereinander leere Lücken (38) aufweisen, die geeignet sind, ein Körperfluid hindurchgehen zu lassen.

2. Vorrichtung (10) nach Anspruch 1, **dadurch gekennzeichnet, daß** sich der spiralförmige Aufbau (35) des Schaftes (30) bis in die Nähe seines distalen Endes (34) erstreckt, ohne es zu erreichen, wobei der Schaft an diesem Ende (34) mit einem vollen Teil dort endet, wo er gegen das Implantat (100) in Abstützung kommt.

3. Vorrichtung (10) nach einem der vorhergehenden Ansprüche, **dadurch gekennzeichnet, daß** sich der spiralförmige Aufbau (35) des Schaftes (30) bis in die Nähe seines proximalen Endes (32) erstreckt, ohne es zu erreichen, derart, daß dieses proximale Ende eine an dem Längsteil des Schaftes fortlaufend volle Umfangswand aufweist, die außerhalb der äußeren Hülle (20) in der ersten relativen Position des Schaftes und der Hülle liegt.

4. Vorrichtung (10) nach einem der vorhergehenden Ansprüche, **dadurch gekennzeichnet, daß** der Schaft (30) mindestens auf einem Teil seiner Länge eine Folge von Abschnitten (35) mit spiralförmigem Aufbau aufweist, die mit einem Abschnitt (Abschnitten) (36) mit fortlaufend voller Umfangsoberfläche abwechseln.

5. Vorrichtung (10) nach einem der vorhergehenden Ansprüche, **dadurch gekennzeichnet, daß** der Schaft (30) innen mindestens auf dem wesentlichen Teil seiner Länge mit einem Katheter (40) ausgekleidet ist.

6. Vorrichtung (10) nach Anspruch 5, **dadurch gekennzeichnet, daß** sich der Katheter (40) bis über das distale Ende (34) des Schaftes (30) erstreckt und im Abstand von diesem von einem profilierten Ansatzstück (50) derart umgeben ist, daß ein Hohlraum (60) zum Einlegen des Implantates (100) bestimmt wird, der zwischen dem distalen Ende (34) des Schaftes und dem Ansatzstück (50) liegt.

7. , Vorrichtung (10) nach Anspruch 6, **dadurch gekennzeichnet, daß** das distale Ende (44) des Katheters (40) im Inneren des profilierten Ansatzstückes (50) endet und durch einen röhrenförmigen, gegen Verletzungen anti-traumatisierenden Abschnitt (41) verlängert wird, der sich über das konisch zulaufende Ende des Ansatzstückes, (50) erstreckt und wesentlich flexibler als der Katheter (40) ist.

8. Vorrichtung (10) nach einem der vorhergehenden Ansprüche, **dadurch gekennzeichnet, daß** die leere Lücke (38) zwischen zwei Windungen (39) des spiralförmigen Aufbaus (35) des Schaftes (30) in einem nicht komprimierten Zustand axial von diesem eine mittlere axiale Länge von zwischen etwa 0,1 und 1 mm aufweist.

9. Vorrichtung nach einem der vorhergehenden Ansprüche, **dadurch gekennzeichnet, daß** die Ganghöhe der Windungen (39) des spiralförmigen Aufbaus (35) des Schaftes (30) zwischen etwa 1 und 5 mm beträgt.

## Claims

1. Device (10) for introducing an implant (100) into an anatomical duct (V), said device (10) comprising:
- a flexible external sheath (20) having a distal end (24) and a proximal end (22),
- a flexible hollow rod (30) having a longitudinal axis (xx'), a length along that axis, a distal end (34) which is to rest against the implant, and a proximal end (32), said rod (30) being slidably mounted inside the sheath (20) over at least a portion of its length,
- the external sheath (20) and the rod (30) being able to slide longitudinally relative to one another in order, in a first relative position, to hold the implant (100) in a first, radially confined, state inside the sheath (20), and, in a second relative position, to enable the implant (100) to be freed outside the sheath (20), in the duct (V), in a second, radially deployed, state,
**characterised in that** the rod (30) comprises, over at least a portion of its length, a helical structure (35) having non-contiguous turns (39) which have, between them, empty interstices (38) capable of allowing a body fluid to pass through.

2. Device (10) according to claim 1, **characterised in that** the helical structure (35) of the rod (30) extends as far as the vicinity of the distal end (34) thereof without reaching it, the rod being terminated at that end (34), at the site where it rests against the implant (100), by an uninterrupted portion.

3. Device (10) according to either of the preceding claims, **characterised in that** the helical structure (35) of the rod (30) extends as far as the vicinity of the proximal end (32) thereof without reaching it, so that this proximal end has a continuous peripheral wall over the portion of the length of the rod that is situated outside the external sheath (20) in said first relative position of the rod and the sheath.

4. Device (10) according to any one of the preceding claims, **characterised in that** the rod (30) comprises, over at least a portion of its length, a series of portions (35) having a helical structure which alternate with (a) portion(s) (36) having a continuous peripheral surface.

5. Device (10) according to any one of the preceding claims, **characterised in that** the rod (30) is lined internally, over at least most of its length, with a catheter (40) .

6. Device (10) according to claim 5, **characterised in that** the catheter (40) extends to beyond the distal end (34) of the rod (30) and is surrounded, at a distance therefrom, by an end member (50) which is shaped in such a manner as to define a cavity (60) for loading the implant (100), which cavity is located between the distal end (34) of the rod and said end member (50).

7. Device (10) according to claim 6, **characterised in that** the distal end (44) of the catheter (40) terminates inside the shaped end member (50) and is extended by an anti-traumatising tubular portion (41) which extends beyond the tapered end of the end member (50) and which is more flexible than said catheter (40).

8. Device (10) according to any one of the preceding claims, **characterised in that** the empty interstice (38) between two turns (39) of the helical structure (35) of the rod (30) has, when the helical structure (35) is in a state in which it is not compressed axially, an average axial length of approximately from 0.1 to 1 millimetre.

9. Device according to any one of the preceding claims, **characterised in that** the pitch of the turns (39) of the helical structure (35) of the rod (30) is approximately from 1 to 5 millimetres.
